# EUROPEAN PATENT SPECIFICATION

(11) **EP 4 190 959 B1**
(45) Date of publication and mention of the grant of the patent: **31.12.2025**
(21) Application number: 21859915.7
(22) Date of filing: 09.07.2021
(51) Int. Cl.: A61L 2/10, H02K 35/02, F21V 33/00, D06F 17/10, D06F 37/12, H02K 7/18, D06F 39/00, D06F 23/04

(54) **CLOTHING TREATMENT DEVICE**
KLEIDUNGSBEHANDLUNGSVORRICHTUNG
DISPOSITIF DE TRAITEMENT DE VÊTEMENTS

(30) Priority: 31.08.2020 CN 202010898189
(43) Date of publication of application: 07.06.2023
(73) Proprietor: CHONGQING HAIER WASHING MACHINE CO., LTD., Chongqing, 400026 (CN); Haier Smart Home Co., Ltd., Qingdao, Shandong 266101 (CN)
(72) Inventor: ZHAO, Zhiqiang, Qingdao, Shandong 266101 (CN); XU, Sheng, Qingdao, Shandong 266101 (CN); WANG, Dejun, Qingdao, Shandong 266101 (CN)
(74) Representative: Pellengahr, Maximilian Rudolf
(86) International application number: PCT/CN2021/105400
(87) International publication number: WO 2022/042075

(56) References cited:
- CN-A- 106 012 406
- CN-A- 106 012 406
- CN-A- 112 080 901
- CN-U- 206 739 072
- CN-U- 211 112 730
- CN-U- 211 112 730
- CN-U- 211 112 754
- CN-U- 211 112 754
- CN-U- 212 247 526
- KR-A- 20200 092 043
- US-A1- 2014 355 247

## Description

### FIELD OF THE INVENTION

The present invention belongs to the technical field of clothing treatment apparatus, and specifically provides a clothing treatment apparatus.

### BACKGROUND OF THE INVENTION

In the process of washing clothing by a washing machine, if an outdoor environment is dark, for example, when the washing machine is arranged in a corner with insufficient light, it is very difficult for the operator to see a washing condition of the clothing inside the washing machine. Especially for some special clothing, such as woolen sweater, the material of the clothing itself is fragile and cannot withstand a large beating force. In the washing process of the clothing, the operator often wants to see clearly the specific washing status of the clothing inside the washing machine so as to adjust washing parameters, thus enabling the washing machine to adapt to the washing of different clothing.

In the prior art, since an interior of the washing machine is rotating, it is very difficult to install an electric lamp on an inner cylinder, and it is also necessary to separately arrange an external power supply for the light emitting lamp. In view of this, the washing machines in the prior art are usually not provided with a light source in the cylinder. It is not that those skilled in the art do not want to set it, but it is because arranging the external power supply separately for the light emitting lamp is too difficult, and the cost is too high.

At present, an electric brush can be selected in the prior art to realize the transmission of electric energy between a moving object and a stationary object. However, due to the large amount of water inside the washing machine itself, the possibility of electric leakage will be greatly increased when using the electric brush for transmission. Obviously, it is not feasible to use the electric brush for transmission on the washing machine, which is a device that attaches great importance to safety. The most advanced technologies include those similar to wireless charging technology, but such technologies need a built-in rechargeable battery, and the overall cost is expensive. In the event of cost control, it is obviously also not suitable to direct apply such technologies to the field of washing machine for illumination.

Chinese utility model patent CN211112730U discloses a washing machine including an electric generation device. The electric generation device includes a first power generation assembly fixed on an inner tub or an output shaft of a motor, a second power generation assembly fixed on an outer tub and corresponding to the first power generation assembly, and a lighting lamp fixed on the inner tub and electrically connected to the first power generation assembly or the second power generation assembly. The washing machine is configured to generate electric power based on the relative rotation between the outer tub and inner tub, or between the outer tub and the output shaft of the motor, and then supply the electric power to the lighting lamp. However, there are no protection parts for the power generation assemblies, so they are easily damaged and the maintenance cost should be high.

Accordingly, there is a need for a new clothing treatment apparatus in the art to solve the problem that it is difficult to arrange an external power supply due to internal rotation of the existing clothing treatment apparatuses.

### SUMMARY OF THE INVENTION

In order to solve the above problem in the prior art, that is, to solve the problem that the electric generation device in washing machine is easily be damaged, the present invention is set out as the appended set of claims.

It can be understood by those in the art that in the technical solutions of the present disclosure, the clothing treatment apparatus includes a rotary part and a stationary part, and the rotary part is rotatably arranged in the stationary part; the clothing treatment apparatus further includes a magnet supply module, a magnet induction power generation module and a light emitting lamp; the magnet induction power generation module is fixed on one of the rotary part or the stationary part, and the magnet supply module is fixed on the other of the rotary part or the stationary part at a position corresponding to that of the magnet induction power generation module; the magnet induction power generation module includes a first housing, a coil and a magnetic conductor; the first housing includes a barrel and a barrel cover, the barrel is fixed on the rotary part or the stationary part, and the coil is fixed on an inner side of the barrel; the magnetic conductor is fixed on an inner side of the coil and is arranged corresponding to the magnet supply module, and the barrel cover encloses the coil and the magnetic conductor inside the barrel; the light emitting lamp is electrically connected with the coil and is fixed on the first housing, or fixed on the rotary part or the stationary part together with the magnet induction power generation module; the magnet supply module generates a magnetic field, and when the rotary part rotates relative to the stationary part, the magnet induction power generation module and the magnet supply module reciprocate in a path close to or away from each other, thus generating electric energy internally, and further supplying power for the light emitting lamp.

Through the above arrangement, the clothing treatment apparatus of the present disclosure can generate electric energy through the relative movement of the magnet supply module and the magnet induction power generation module, so that the light emitting lamp lights up. The clothing treatment apparatus of the present disclosure does not need an external power supply, and it only makes the magnetic field be close to and away from the magnetic conductor so that the magnetic conductor generates a varying magnetic field; thus, the fixed coil can also cut magnetic lines of force to generate electric energy, so that it is unnecessary to connect a power supply into the interior, and thus there will be no electric wires near the rotary part, thus solving the problem that it is difficult for the clothing treatment apparatus to separately arrange an external power supply for the light emitting lamp.

### BRIEF DESCRIPTION OF THE DRAWINGS

The clothing treatment apparatus of the present disclosure will be described below with reference to the accompanying drawings and in connection with a pulsator washing machine. In the drawings:
FIG. 1 is a schematic view showing an internal structure between a pulsator and an inner tub of a pulsator washing machine;
FIG. 2 is a schematic view showing the position of a magnet induction power generation module installed on the pulsator;
FIG. 3 is an exploded view of the magnet induction power generation module and a magnet supply module; and
FIG. 4 shows the magnet induction power generation module and the magnet supply module after completion of assembly.

### List of reference signs:

1: magnet supply module; 11: permanent magnet; 12: third housing; 2: magnet induction power generation module; 21: first housing; 211: barrel; 2111: light output hole; 2112: transparent glass; 212: barrel cover; 22: second housing; 221: winding part; 222: hollow hole; 223: lampstand; 23: coil; 24: magnetic conductor; 3: light emitting lamp; 4: rotary part; 5: stationary part.

### DETAILED DESCRIPTION OF THE EMBODIMENT(S) OF THE INVENTION

Preferred embodiments of the present disclosure will be described below with reference to the accompanying drawings. It should be understood by those skilled in the art that these embodiments are only used to explain the technical principle of the present disclosure, and are not intended to limit the scope of protection of the present disclosure. Those skilled in the art can make adjustments thereto as required so as to adapt to specific application scenes. For example, although an illumination lamp is used as an example of the light emitting lamp in the specification for description, obviously, the light emitting lamp of the present disclosure can also be other functional lamps such as an ultraviolet sterilization lamp or a mosquito killing lamp, so as to meet other needs of users.

It should be noted that in the description of the present disclosure, terms indicating directional or positional relationships, such as "center", "above", "below", "left", "right", "vertical", "horizontal", "inner", "outer" and the like, are based on the directional or positional relationships shown in the accompanying drawings. They are only used for ease of description, and do not indicate or imply that the device or element must have a specific orientation, or be constructed or operated in a specific orientation; therefore, they should not be considered as limitations to the present disclosure. In addition, terms "first", "second" and "third" are only used for descriptive purposes, and should not be interpreted as indicating or implying relative importance.

In addition, it should also be noted that in the description of the present disclosure, unless otherwise clearly specified and defined, terms "install", "connect" and "connection" should be understood in a broad sense; for example, the connection may be a fixed connection, or may also be a detachable connection, or an integral connection; it may be a mechanical connection, or an electrical connection; it may be a direct connection, or an indirect connection implemented through an intermediate medium, or it may be internal communication between two elements. For those skilled in the art, the specific meaning of the above terms in the present disclosure can be interpreted according to specific situations.

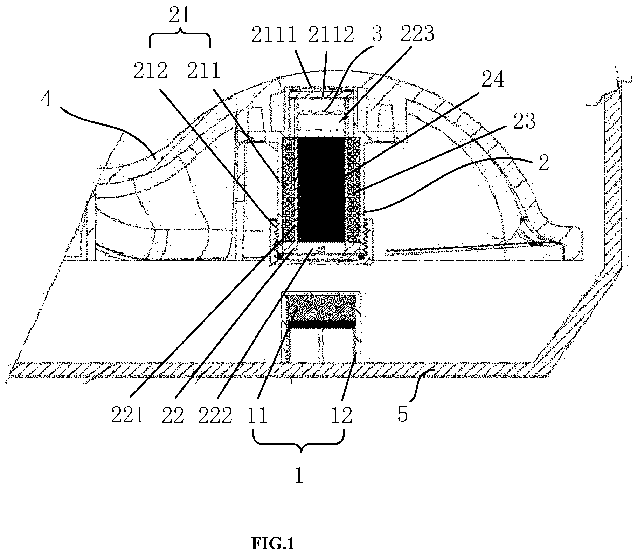

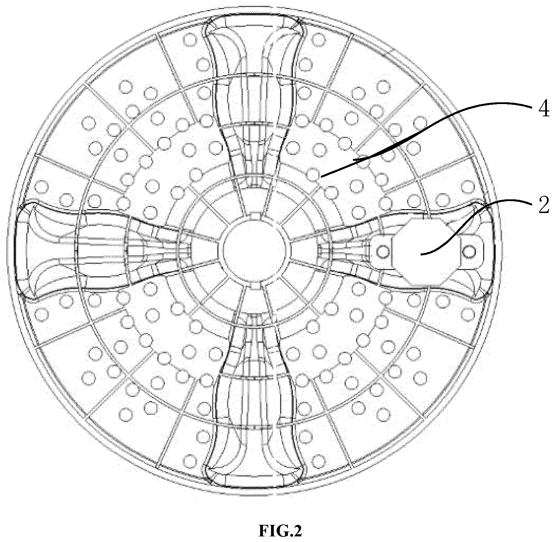

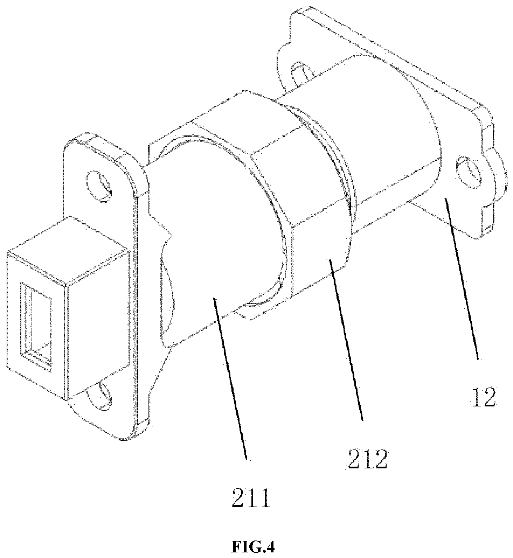

Referring to FIGS. 1 to 4, in order to solve the problem that it is difficult for the existing clothing treatment apparatuses to separately arrange an external power supply for the light emitting lamp, in a possible embodiment, the clothing treatment apparatus of the present disclosure includes a rotary part 4 and a stationary part 5, and the rotary part 4 is rotatably arranged in the stationary part 5; the clothing treatment apparatus further includes a magnet supply module 1, a magnet induction power generation module 2 and a light emitting lamp 3; the magnet induction power generation module 2 is fixed on the rotary part 4, and the magnet supply module 1 is fixed on the stationary part 5 at a position corresponding to that of the magnet induction power generation module 2; of course, the positions of the magnet induction power generation module 2 and the magnet supply module 1 can be reversed, as long as a relative movement between the two can be achieved to realize power generation, thus further supplying power for the light emitting lamp 3.

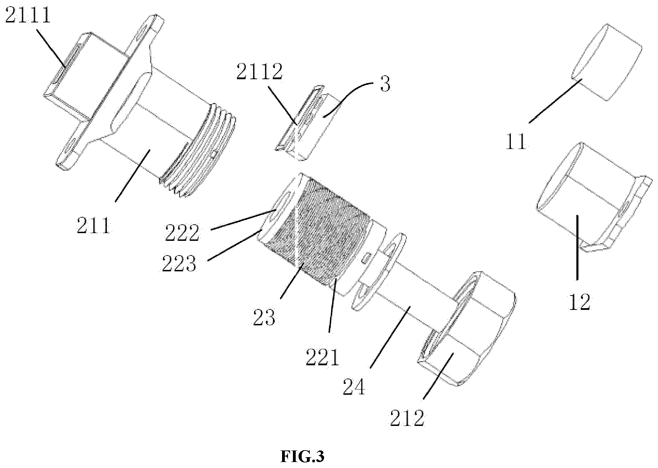

As shown in FIGS. 1 and 3, the magnet supply module 1 includes a permanent magnet 11 and a third housing 12; the third housing 12 is fixed on an inner tub of the stationary part 5, and the permanent magnet 11 is fixed on the third housing 12, thus generating a lasting magnetic field. Of course, the magnet supply module 1 can also have only one permanent magnet 11, which is directly embedded and fixed on the inner tub of the stationary part 5. Alternatively, the magnet supply module 1 can also be an electromagnet; since the magnet supply module 1 is fixed on the stationary part 5, the stationary part 5 alone can be easily connected to the power supply, so the electromagnet is also feasible.

Referring further to FIGS. 1 and 3, the magnet induction power generation module 2 includes a first housing 21, a coil 23 and a magnetic conductor 24. The first housing 21 includes a barrel 211 and a barrel cover 212. The barrel 211 is fixed on a pulsator of the rotary part 4, the coil 23 is fixed on an inner side of the barrel 211, and the magnetic conductor 24 is fixed on an inner side of the coil 23 and is arranged corresponding to the magnet supply module 1; that is, when the pulsator rotates, the magnetic conductor 24 can enter and leave the magnetic field of the magnet supply module 1 and generate an induction magnetic field by itself. The barrel cover 212 encloses the coil 23 and the magnetic conductor 24 inside the barrel 211, and the light emitting lamp 3 is electrically connected with the coil 23 and fixed on the first housing 21. The magnetic conductor 24 can be an iron core; of course, it can also be other members or devices, as long as it is an object that can generate a varying magnetic field by itself as the permanent magnet 11 becomes close to or away from the magnetic conductor 24.

The overall working process is described as follows: when the pulsator starts to rotate, the magnet induction power generation module 2 will also rotate therewith, whereas the permanent magnet 11 is fixed on the inner tub and cannot move. At this time, the magnet induction power generation module 2 will become close to the permanent magnet 11 once and away from the permanent magnet 11 once every time the pulsator rotates by one turn; correspondingly, the magnetic conductor 24 will increase its own magnetic field when it is close to the permanent magnet 11, and will decrease its own magnetic field when it is away from the permanent magnet 11. Each time the pulsator rotates by one turn, the magnetic conductor 24 will complete one time of change in the magnitude of the magnetic field. The constant change of the magnetic field will cause the magnetic lines of force to move relative to the coil 23, which realizes cutting of the magnetic lines of force by the coil 23, thus generating the current and lighting up the light emitting lamp 3.

The advantage of the above arrangement is that it is simple and easy to implement, and it can realize the illumination inside the pulsator washing machine without providing a separate wiring, thus solving the problem of wiring for an external power supply. The design of the permanent magnet 11 and the magnetic conductor 24 elaborately solves the conduction between magnetic fields, making the overall structure simpler and more feasible. The split arrangement of the barrel 211 and the barrel cover 212 enables the operator to assemble very easily, and in the subsequent maintenance process, after the pulsator is removed, it is also very easy to replace the entirety of the combination of the barrel 211 and the barrel cover 212 by unscrewing or separately replace damaged parts such as the coil 23 and the magnetic conductor 24 in the barrel 211, thus reducing the subsequent maintenance cost. Moreover, according to the design of the barrel 211 and the barrel cover 212 of the present disclosure, convenient installation can be realized by only adding a few threaded holes on the basis of the original pulsator structure design. There is no major change in the overall structure of the washing machine, which makes the clothing treatment apparatus of the present disclosure compatible with the original production line in the production process without making major changes. Since there is very little modification to the original structure, an optional installation can be provided to the users to achieve price classification, thus enabling the users to have more choices.

With continued reference to FIGS. 1 and 3 below, in another possible embodiment, the magnet induction power generation module 2 further includes a second housing 22, which is fixed on the inner side of the barrel 211; the coil 23 is wound on an outer side of the second housing 22, the magnetic conductor 24 is fixed on an inner side of the second housing 22, and the barrel cover 212 seals the second housing 22 in the barrel 211. The barrel cover 212 is snap-fit with the second housing 22, and the barrel cover 212 is connected with the barrel 211 through threads. The second housing 22 includes a winding part 221, a hollow hole 222 and a lampstand 223. The coil 23 is wound on the winding part 221, the magnetic conductor 24 is arranged in the hollow hole 222, and the light emitting lamp 3 is arranged in the lampstand 223. The barrel 211 is provided with a light output hole 2111, and the inner side of the barrel 211 is further provided with a transparent glass 2112. The transparent glass 2112 covers the light output hole 2111, and a top end of the lampstand 223 abuts against the transparent glass 2112. The transparent glass 2112 can be quartz glass, transparent plastic glass or other glass that is advantageous for light transmission and has a sufficient strength, as long as it can ensure smooth passage of light.

The above arrangement has the following advantage: the second housing 22 is arranged to have the light emitting lamp 3, the coil 23 and the magnetic conductor 24 all integrated thereon; then the second housing 22 is snap-fit on the barrel cover 212 (of course, it can also be connected through other conventional connection means such as thread), and the barrel cover 212 and the connected second housing 22 are inserted together into the barrel 211 before being connected and fixed by threads, which will make the roles of the barrel 211, the barrel cover 212 and the second housing 22 completely independent and separate from each other. The barrel 211 and the barrel cover 212 are only responsible for the overall fixation and waterproofing. The second housing 22 has the light emitting lamp 3, the coil 23 and the magnetic conductor 24 integrated thereon, which can be completely independently subcontracted to different manufacturers in the early processing stage. In the subsequent maintenance stage, if it is only the internal parts such as the light emitting lamp 3 that are damaged, the overall removal and replacement of the internal parts can also be easily completed, and there is no need to replace the external barrel 211 and barrel cover 212. At the same time, since the second housing 22 is inserted into the barrel 211, a waterproof structure, such as a sealing ring, can still be arranged between the second housing 22 and the barrel 211 to achieve multilayer waterproof, so that the overall structure has better waterproof performance and longer service life.

To sum up, due to the relative movement of the rotary part 4 and the stationary part 5, wiring is difficult in the prior art, whereas the present disclosure elaborately takes advantage of the relative movement of the rotary part 4 and the stationary part 5 and the change of magnetic field caused by the conduction of the magnet, so that the coil 23 in the magnet induction power generation module 2 can cut the magnetic lines of force to realize power generation and supply power for the light emitting lamp, which elaborately converts the obstruction into a power source and dispenses with the need for wiring. In addition, in order to save processing cost and later maintenance cost and rationalize the structure layout, the barrel 211 and the barrel cover 212 are designed to be waterproof, the internal structure is designed to be easily disassembled and assembled, and the second housing 22 is designed to be able to integrate all of the coil 23, the magnetic conductor 24 and the light emitting lamp 3 together to realize the overall insertion and removal into/from the barrel 211, making their maintenance steps as convenient as the daily removal of household bulbs. Due to the high integration of the internal structure, the combined parts of the second housing 22 with the coil 23, the magnetic conductor 24 and the light emitting lamp 3 can also achieve the unification of the inner core. It is only necessary to adjust the barrel 211 and the barrel cover 212 according to different structure models of the washing machine to achieve the universality of the internal power generation structure, and the internal structures subsequently provided for the market can be unified, which can achieve great cost reduction.

It should be noted that the above embodiments are only used to illustrate the principle of the present disclosure and are not intended to limit the scope of protection of the present disclosure. Without departing from the principle of the present disclosure, those skilled in the art can adjust the above structure so that the present disclosure can be applied to more specific application scenes.

For example, in an alternative embodiment, the light emitting lamp 3 can be an illumination lamp such as an LED lamp, or an ultraviolet sterilization lamp for sterilization purpose, or a combination of the two for function switching, or other functional lamps, such as a mosquito killing lamp, all of which do not deviate from the principle of the present disclosure, and therefore will fall within the scope of protection of the present disclosure.

For example, in another alternative embodiment, the positions for fixing the light emitting lamp 3 are various. For example, when the specific embodiment is the structure in FIG. 1, the light emitting lamp 3 can be fixed on either of the first housing 21, the second housing 22 or the pulsator. Correspondingly, if the magnet induction power generation module 2 is arranged on the inner tub, the magnet supply module 1 will be arranged on the pulsator; at this time, the light emitting lamp 3 can also be arranged on the inner tub of the stationary part 5. Those skilled in the art can select suitable installation positions according to actual needs, all of which do not deviate from the principle of the present disclosure, and therefore fall within the scope of protection of the present disclosure.

For example, in another alternative embodiment, although only one magnet induction power generation module 2 and one magnet supply module 1 are shown exemplarily in FIG. 1, it is obvious that the numbers of the two can be set at will, and they do not have to be set with the numbers thereof corresponding to each other. For example, three magnet supply modules 1 can be arranged at a bottom of the inner tub in a circular path, but five magnet induction power generation modules 2 can be arranged on the pulsator. As the pulsator rotates, it is obvious that the magnet induction power generation modules 2 can still generate electricity to light up the light emitting lamp 3. These do not deviate from the principle of the present disclosure, so they will fall within the scope of protection of the present disclosure.

Finally, it should be noted that although the present disclosure is described by taking a pulsator washing machine as an example, the clothing treatment apparatus of the present disclosure can obviously be other apparatuses. For example, it can also be a drum washing machine, etc.; correspondingly, the rotary part 4 is an inner cylinder, and the stationary part 5 is an outer cylinder; the fixing method thereof is also similar to the pulsator washing machine, and the principle is the same, so a repeated description will be omitted herein.

It should be noted that the innovation of the present disclosure further lies in that when facing such problems, the first condition for those skilled in the art is to arrange a wire on the periphery of the rotary part 4. However, as long as the wire is arranged near the periphery of the rotary part 4, there will be more wires passing to the vicinity of the rotary part 4 through an operation panel; especially for the drum washing machine, these wires are usually clamped on the long-term stationary housing or outer cylinder on the outer side the rotary part 4 through buckles. Such a method has no significant influence in a short term. However, since the washing machine is an electronic product for long term use and has a long service life, circuit safety and quality problems thereof cannot be ignored. Under long-term vibration conditions, the buckles usually fall off, which makes it impossible for the wires to fully fit the outer side any longer. Once the wires are rolled into rotation by the rotary part 4, they will cause serious electric leakage accidents and even fire. In view of this, although it is very necessary to illuminate the rotary part 4 of the drum washing machine, for safety reasons, those skilled in the art have not yet developed a wiring method with high safety performance to solve this problem. In view of the facts that the light emitting lamp 3 itself does not need much electric energy to maintain its brightness, and that the more wires arranged outside the rotary part 4, the higher the risk, the inventor creatively proposed the above solution to solve the power supply problem of the light emitting lamp 3. After many tests, the inventor found that the electric energy generated by cutting the magnetic lines of force by the coil 23 is enough to maintain the illumination of the light emitting lamp 3. This method does not need wiring on the outer side of the rotary part 4, thus solving the problem that more wires are easily rolled into the rotary part 4, saving electric energy, and also avoiding the problem that the electrical connection between the rotating drum and the stationary external power supply is difficult, which provides a new direction for solving the problem of internal illumination of the washing machine.

Hitherto, the technical solutions of the present disclosure have been described in connection with the preferred embodiments shown in the accompanying drawings, but it is easily understood by those skilled in the art that the scope of protection of the present disclosure is obviously not limited to these specific embodiments. Without departing from the principles of the present disclosure, those skilled in the art can make equivalent changes or replacements to relevant technical features, and all the technical solutions after these changes or replacements will fall within the scope of protection of the claims.

## Claims

1. A clothing treatment apparatus, comprising a rotary part (4) ) and a stationary part (5), the rotary part (4) being rotatably arranged in the stationary part (5), wherein the clothing treatment apparatus further comprises a magnet supply module (1), a magnet induction power generation module (2) and a light emitting lamp (3);
the magnet induction power generation module (2) is fixed on one of the rotary part (4) or the stationary part (5), and the magnet supply module (1) is fixed on the other of the rotary part (4) or the stationary part (5) at a position corresponding to that of the magnet induction power generation module (2);
the magnet induction power generation module (2) comprises a first housing (21), a coil (23) and a magnetic conductor (24); the first housing (21) comprises a barrel (211) and a barrel cover (212), the barrel (211) is fixed on the rotary part (4) or the stationary part (5), and the coil (23) is fixed on an inner side of the barrel (211); the magnetic conductor (24) is fixed on an inner side of the coil (23) and is arranged corresponding to the magnet supply module (1), and the barrel cover (212) encloses the coil (23) and the magnetic conductor (24) inside the barrel (211);
the light emitting lamp (3) is electrically connected with the coil (23) and is fixed on the first housing (21), or fixed on the rotary part (4) or the stationary part (5) together with the magnet induction power generation module (2); and
the magnet supply module (1) generates a magnetic field, and when the rotary part (4) rotates relative to the stationary part (5), the magnet induction power generation module (2) and the magnet supply module (1) reciprocate in a path close to or away from each other, thus generating electric energy internally, and further supplying power for the light emitting lamp (3);
wherein the magnet induction power generation module (2) further comprises a second housing (22), which is fixed on the inner side of the barrel (211); the coil (23) is wound on an outer side of the second housing (22), the magnetic conductor (24) is fixed on an inner side of the second housing (22), and the barrel cover (212) seals the second housing (22) in the barrel (211).

2. The clothing treatment apparatus according to claim 1, wherein the barrel cover (212) and the barrel (211) are connected by threads; and/or
the barrel cover (212) is snap-fit with the second housing (22).

3. The clothing treatment apparatus according to claim 1, wherein the second housing (22) comprises a winding part (221), a hollow hole (222) and a lampstand (223); the coil (23) is wound on the winding part (221), the magnetic conductor (24) is arranged in the hollow hole (222), and the light emitting lamp (3) is arranged in the lampstand (223).

4. The clothing treatment apparatus according to claim 3, wherein the barrel (211) is provided with a light output hole (2111), and the inner side of the barrel (211) is further provided with a transparent glass (2112); the transparent glass (2112) covers the light output hole (2111), and a top end of the lampstand (223) abuts against the transparent glass (2112).

5. The clothing treatment apparatus according to claim 1, wherein the magnetic conductor (24) is an iron core.

6. The clothing treatment apparatus according to claim 1, wherein the magnet supply module (1) comprises a permanent magnet (11).

7. The clothing treatment apparatus according to claim 6, wherein the magnet supply module (1) further comprises a third housing (12), and the permanent magnet (11) is fixed inside the third housing (12).

8. The clothing treatment apparatus according to claim 1, wherein the magnet supply module (1) and/or the magnet induction power generation module (2) are each provided in plural; and/or
the light emitting lamp (3) is an ultraviolet sterilization lamp or an LED illumination lamp.

9. The clothing treatment apparatus according to claim 1, wherein the clothing treatment apparatus is a pulsator washing machine; correspondingly, the rotary part (4) is a pulsator, and the stationary part (5) is an inner tub; and/or
the clothing treatment apparatus is a drum washing machine; correspondingly, the rotary part (4) is an inner cylinder, and the stationary part (5) is an outer cylinder.

## Patentansprüche

1. Eine Bekleidungsbehandlungsanlage, umfassend einen rotierenden Teil (4) und einen stationären Teil (5), wobei der rotierende Teil (4) drehbar im stationären Teil (5) angeordnet ist, wobei das Bekleidungsbehandlungsgerät ferner ein Magnetversorgungsmodul (1), ein Magnetinduktionsspannungsmodul (2) und eine lichtemittierende Lampe (3) umfasst;
Das Magnetinduktion-Generator-Modul (2) ist auf einem der rotierenden Teil (4) oder des festen Teils (5) befestigt, und das Magnetversorgungsmodul (1) ist auf dem anderen der rotierenden Teil (4) oder des festen Teils (5) an einer Position befestigt, die der Position des Magnetinduktion-Generator-Moduls (2) entspricht.
Das Magnetinduktion-Generatorelement (2) umfasst ein erstes Gehäuse (21), eine Spule (23) und einen magnetischen Leiter (24); das erstes Gehäuse (21) besteht aus einem Zylinder (211) und einer Zylinderabdeckung (212), wobei der Zylinder (211) auf der rotierenden Teil (4) oder der festen Teil (5) befestigt ist und die Spule (23) an der Innenseite des Zylinders (211) befestigt ist; der magnetische Leiter (24) ist an der Innenseite der Spule (23) befestigt und entsprechend dem Magnetversorgungselement (1) angeordnet, wobei die Zylinderabdeckung (212) die Spule (23) und den magnetischen Leiter (24) im Inneren des Zylinders (211) umschließt.
Die lichtemittierende Lampe (3) ist elektrisch mit der Spule (23) verbunden und befestigt auf der ersten Gehäuse (21), oder befestigt zusammen mit dem magnetinduktiven Stromerzeugungsmodul (2) auf der rotierenden Teil (4) oder der stationären Teil (5); und
Das Magnetversorgungsmodul (1) erzeugt ein Magnetfeld, und wenn die rotierende Komponente (4) relativ zur stationären Komponente (5) rotiert, pendeln das Magnetinduktionsenergieerzeugungsmodul (2) und das Magnetversorgungsmodul (1) entlang einer Bahn auf- oder abwärts, wodurch elektrische Energie intern erzeugt wird und weiterhin Strom für die Leuchtlampe (3) bereitgestellt wird.
wobei das Magnetinduktionsgleichstromerzeugungsmodul (2) weiterhin ein zweites Gehäuse (22) umfasst, das auf der Innenseite des Zylinders (211) befestigt ist; die Spule (23) ist auf der Außenseite des zweiten Gehäuses (22) gewickelt, der Magnetleiter (24) ist auf der Innenseite des zweiten Gehäuses (22) befestigt, und die Zylinderabdeckung (212) versiegelt das zweite Gehäuse (22) im Zylinder (211).

2. Die Bekleidungsbehandlungsanlage nach Anspruch 1, **dadurch gekennzeichnet, dass** die Trommelschale (212) und die Trommel (211) durch Gewinde verbunden sind; und/oder
Die Fassabdeckung (212) ist schnappverschraubt mit dem zweiten Gehäuse (22).

3. Die Bekleidungsbehandlungsanlage nach Anspruch 1, **dadurch gekennzeichnet, dass** die zweite Gehäuse (22) eine Wickelteil (221), eine Hohlbohrung (222) und einen Leuchter (223) umfasst; die Spule (23) ist auf dem Wickelteil (221) gewickelt, der magnetische Leiter (24) ist in der Hohlbohrung (222) angeordnet, und die lichtemittierende Lampe (3) ist im Leuchter (223) angeordnet.

4. Die Bekleidungsbehandlungsanlage nach Anspruch 3, wobei der Zylinder (211) mit einem Lichtausgangsloch (2111) ausgestattet ist und die Innenseite des Zylinders (211) weiterhin mit einem transparenten Glas (2112) versehen ist; das transparente Glas (2112) bedeckt das Lichtausgangsloch (2111), und die obere Endseite der Lampe (223) liegt gegen das transparente Glas (2112) an.

5. Die Bekleidungsbehandlungsanlage nach Anspruch 1, wobei der magnetische Leiter (24) ein Eisenkern ist.

6. Die Bekleidungsbehandlungsanlage nach Anspruch 1, **dadurch gekennzeichnet, dass** das Magnetversorgungsmodul (1) ein Permanentmagnet (11) umfasst.

7. Die Bekleidungsbehandlungsanlage nach Anspruch 6, **dadurch gekennzeichnet, dass** das Magnetversorgungsmodul (1) weiterhin ein drittes Gehäuse (12) umfasst und der Permanentmagnet (11) innerhalb des dritten Gehäuses (12) fixiert ist.

8. Die Bekleidungsbehandlungsanlage nach Anspruch 1, **dadurch gekennzeichnet, dass** das Magnetversorgungsmodul (1) und/oder das Magnetinduktionsspannungsmodul (2) jeweils mehrfach vorgesehen sind; und/oder
Die lichtemittierende Lampe (3) ist eine UV-Bestrahlungslampe oder eine LED-Belichtungslampe.

9. Die Bekleidungsbehandlungsanlage nach Anspruch 1, wobei die Bekleidungsbearbeitungsanlage eine Pulsator-Waschmaschine ist; entsprechend ist der rotierende Teil (4) ein Pulsator und der feste Teil (5) ein Innenwanne; und/oder
Das Bekleidungsbehandlungsgerät ist eine Trommelwäsche; entsprechend ist der rotierende Teil (4) ein innerer Zylinder, und der stationäre Teil (5) ist ein äußerer Zylinder.

## Revendications

1. Un appareil de traitement de vêtements, comprenant une partie rotative (4) et une partie stationnaire (5), la partie rotative (4) étant disposée de manière rotative dans la partie stationnaire (5), dans lequel l'appareil de traitement de vêtements comprend en outre un module d'alimentation magnétique (1), un module de génération d'énergie par induction magnétique (2) et une lampe émissive de lumière (3) ;
le module de génération de puissance par induction magnétique (2) est fixé sur l'une des parties rotatives (4) ou sur la partie fixe (5), et le module d'alimentation magnétique (1) est fixé sur l'autre des parties rotatives (4) ou sur la partie fixe (5) à une position correspondant à celle du module de génération de puissance par induction magnétique (2).
Le module de génération d'énergie par induction magnétique (2) comprend un premier boîtier (21), une bobine (23) et un conducteur magnétique (24) ; le premier boîtier (21) comprend un cylindre (211) et un couvercle de cylindre (212), le cylindre (211) étant fixé sur la partie rotative (4) ou la partie stationnaire (5), et la bobine (23) étant fixée sur le côté intérieur du cylindre (211) ; le conducteur magnétique (24) est fixé sur le côté intérieur de la bobine (23) et est disposé en correspondance avec le module d'alimentation magnétique (1), et le couvercle de cylindre (212) enferme la bobine (23) et le conducteur magnétique (24) à l'intérieur du cylindre (211) ;
la lampe émissive de lumière (3) est connectée électriquement à la bobine (23) et est fixée sur le premier boîtier (21), ou fixée sur la partie rotative (4) ou la partie fixe (5) avec le module de génération d'énergie par induction magnétique (2) ; et
Le module d'alimentation magnétique (1) génère un champ magnétique, et lorsque la partie rotative (4) tourne par rapport à la partie fixe (5), le module de génération d'énergie par induction magnétique (2) et le module d'alimentation magnétique (1) se déplacent alternativement le long d'une trajectoire proche ou éloignée l'un de l'autre, générant ainsi de l'énergie électrique intérieurement et fournissant en outre de l'alimentation pour la lampe à émission lumineuse (3).
où le module de génération d'énergie par induction magnétique (2) comprend en outre un deuxième boîtier (22) fixé sur la face intérieure du cylindre (211) ; la bobine (23) est enroulée sur la face extérieure du deuxième boîtier (22), le conducteur magnétique (24) est fixé sur la face intérieure du deuxième boîtier (22), et le couvercle du cylindre (212) scelle le deuxième boîtier (22) dans le cylindre (211).

2. L'appareil de traitement des vêtements selon la revendication 1, dans lequel le couvercle de tambour (212) et le tambour (211) sont connectés par des filets ; et/ou
Le couvercle du baril (212) est fixé par encoche sur la deuxième enveloppe (22).

3. Appareil de traitement des vêtements selon la revendication 1, dans lequel le deuxième boîtier (22) comprend une partie de bobinage (221), un trou creux (222) et un support de lampe (223) ; la bobine (23) est enroulée sur la partie de bobinage (221), le conducteur magnétique (24) est disposé dans le trou creux (222), et la lampe émissive (3) est disposée dans le support de lampe (223).

4. L'appareil de traitement des vêtements selon la revendication 3, dans lequel le cylindre (211) est équipé d'un orifice de sortie de lumière (2111), et le côté intérieur du cylindre (211) est en outre équipé d'un verre transparent (2112) ; le verre transparent (2112) recouvre l'orifice de sortie de lumière (2111), et l'extrémité supérieure de la lampe (223) est en contact avec le verre transparent (2112).

5. L'appareil de traitement de vêtements selon la revendication 1, dans lequel le conducteur magnétique (24) est un noyau en fer.

6. L'appareil de traitement de vêtements selon la revendication 1, dans lequel le module d'alimentation magnétique (1) comprend un aimant permanent (11).

7. L'appareil de traitement des vêtements selon la revendication 6, dans lequel le module d'alimentation en aimants (1) comprend en outre un troisième boîtier (12), et l'aimant permanent (11) est fixé à l'intérieur du troisième boîtier (12).

8. L'appareil de traitement des vêtements selon la revendication 1, dans lequel le module d'alimentation magnétique (1) et/ou le module de génération d'énergie par induction magnétique (2) sont chacun fournis en plusieurs exemplaires ; et/ou
la lampe émissive (3) est une lampe de stérilisation ultraviolette ou une lampe d'éclairage LED.

9. L'appareil de traitement des vêtements selon la revendication 1, dans lequel l'appareil de traitement des vêtements est une machine à laver à pulsateur ; de manière correspondante, la partie rotative (4) est un pulsateur, et la partie fixe (5) est un tambour intérieur ; et/ou
L'appareil de traitement des vêtements est une machine à laver à tambour ; par conséquent, la partie rotative (4) est un cylindre intérieur, et la partie fixe (5) est un cylindre extérieur.
